# FASCICULE DE BREVET EUROPEEN

(11) **EP 1 226 417 B1**
(45) Date de publication et mention de la délivrance du brevet: **17.01.2007**
(21) Numéro de dépôt: 00974630.6
(22) Date de dépôt: 02.11.2000
(51) Int. Cl.: G01N 3/10, G01N 33/24, E02D 1/02, E02D 1/04

(54) **PROCEDE ET DISPOSITIF D'ESSAI TRIAXIAL IN SITU**
DREI-ACHSEN-TESTVERFAHREN UND -GERÄT
METHOD AND DEVICE FOR IN SITU TRIAXIAL TEST

(30) Priorité: 04.11.1999 FR 9913792
(43) Date de publication de la demande: 31.07.2002
(73) Titulaire: LABORATOIRE CENTRAL DES PONTS ET CHAUSSEES, 75015 Paris (FR)
(72) Inventeur: REIFFSTECK, Philippe, F-92260 Fontenay aux Roses (FR)
(74) Mandataire: Dronne, Guy
(86) Numéro de dépôt international: PCT/FR2000/003050
(87) Numéro de publication internationale: WO 2001/033194

(56) Documents cités:
- FR-A- 2 212 838
- US-A- 4 649 737
- US-A- 5 435 187

## Description

L'invention a pour objet un procédé pour mesurer in situ les modules de déformation d'un échantillon de sol dans des conditions voisines d'un essai triaxial, et un dispositif pour mettre en oeuvre le procédé.

Traditionnellement, la mesure directe des modules de déformation d'un sol se fait par un procédé dénommé "essai triaxial de révolution". Pour cela, on prélève dans le sol des carottes au moyen d'un carottier, ce qui n'est pas sans incidence sur la qualité de l'échantillon obtenu. Malgré la sophistication des carottiers, il apparaît un remaniement lors de la pénétration de l'étui par la création de voûtes provoquées par le frottement sol-paroi interne de l'étui. Les phases intermédiaires entre le prélèvement de la carotte et l'essai proprement dit provoquent une relaxation des contraintes initiales de l'échantillon. Pour éloigner l'échantillon de cette frontière soupçonnée de remaniement, on taille généralement celui-ci loin des bords extérieurs de la carotte. Cette précaution provoque une importante perte de matière.

L'essai en laboratoire, appelé essai triaxial est un essai homogène qui permet de déterminer par identification les modules de déformation du sol qui sont nécessaires à un calcul en déformation, par exemple de type "éléments finis".

L'essai triaxial consiste à placer l'échantillon à tester, de forme globalement cylindrique, à section circulaire ou carrée, dans une enveloppe cylindrique et dilatable. Cette enveloppe est introduite dans une enceinte pleine de liquide dont la pression peut être réglée, et elle est disposée entre deux pistons qui peuvent exercer des forces sur les faces d'extrémité de l'échantillon. Des appareils de mesure permettent de mesurer les déplacements de la paroi de l'enveloppe, les déplacements des pistons et les pressions dans l'enceinte et dans le sol. La mesure du module de déformation se fait soit en augmentant la pression dans l'enceinte et les forces appliquées sur les pistons et on enregistre alors la variation de volume de l'échantillon, soit en injectant un volume connu de liquide dans l'enceinte et on enregistre dans ce cas la variation de pression qui en résulte dans l'échantillon.

Les brevets US 4 502 338 et US 4 579 003 décrivent des instruments pour tester des échantillons dans des conditions de contraintes triaxiales.

L'essai triaxial permet la mesure directe des modules de déformation. Il permet également de contrôler les conditions de drainage et de déterminer les paramètres d'anisotropie du sol traité.

Il souffre toutefois de plusieurs défauts. Il est d'abord lourd à mettre en oeuvre et par conséquent onéreux, lorsqu'il s'agit d'étudier les sols les plus intacts.

Il nécessite en plus beaucoup de temps, car il faut recréer, par la phase de consolidation, le champ de contrainte initial avant de réaliser le test. Le résultat est en outre susceptible d'être biaisé par un certain nombre d'erreurs, dues aux jeux au niveau des surfaces de contact et à la non-coaxialité dans l'empilage mécanique de la machine d'essai.

Pour éviter certains de ces inconvénients, il a déjà été proposé de réaliser des essais de résistance du sol in situ, au moyen d'outils que l'on dilate dans un trou de forage.

FR 1 596 747 propose ainsi une sonde foreuse pressiométrique qui comporte un corps de sonde muni, sur son pourtour extérieur, d'une membrane souple et dans lequel est disposée une tige creuse, et un outil coupant constitué par un corps de sonde et par un organe désagrégateur disposé dans le corps creux et solidaire de la tige creuse.

Cette sonde autoforeuse permet de faire des mesures au fur et à mesure de l'avancement du forage. Mais l'inconvénient des mesures effectuées avec cette sonde est que l'essai pressiométrique est basé sur l'expansion de la sonde dans une cavité cylindrique ménagée dans un sol de dimension infinie. Or, dans ces conditions, l'état de contrainte engendré dans le sol n'est pas homogène, et donc les modules de déformation ne peuvent être obtenus que par des lois empiriques difficiles à mettre au point.

L'usage de l'essai pressiométrique s'est imposé pour le dimensionnement des fondations. Toutefois, dans le domaine des soutènements et des glissements de terrain, seuls les essais de laboratoire restent les essais de base.

Le but de l'invention est de proposer un procédé et un dispositif qui permettent de créer, in situ, un champ de contrainte homogène dans un volume fini de sol à tester, tout en évitant tout mouvement de l'échantillon de sol à tester avant le début véritable de chaque essai.

Le but est également de proposer un procédé et un dispositif de mesure in situ des modules de déformation d'un échantillon de sol dans les conditions d'un test triaxial de révolution qui puisse être utilisé notamment dans le domaine des soutènements et des glissements de terrain.

Selon l'invention, le procédé est caractérisé par les étapes suivantes :
on enfonce dans le sol un carottier équipé, sur sa face interne, d'une membrane souple,
on évacue les matériaux qui ressortent par l'orifice supérieur du carottier au cours de son enfoncement, afin de former un échantillon de sol à tester dans le carottier,
on immobilise le carottier dans le sol pour procéder à une mesure,
on soumet l'échantillon à des contraintes radiales et axiales en exerçant des efforts de pression sur la face externe de la membrane et sur la face supérieure de l'échantillon et on mesure les déformations subies par l'échantillon.

De préférence, pendant la mesure, on soumet l'échantillon à des contraintes radiales prédéterminées et on fait varier les contraintes axiales jusqu'à la rupture de l'échantillon.

Afin de déterminer les paramètres d'anisotropie du sol, on utilise un carottier à section carrée.

Le dispositif selon l'invention pour la mise en oeuvre du procédé comporte :
un carottier équipé sur sa face interne d'une membrane souple,
des moyens pour enfoncer ledit carottier dans le sol afin de former un échantillon dans ledit carottier,
des moyens pour évacuer les matériaux qui ressortent par l'orifice supérieur dudit carottier au cours de son enfoncement dans le sol,
des moyens pour immobiliser ledit carottier au cours de la mesure,
des moyens pour appliquer une force axiale sur la face supérieure de l'échantillon,
des moyens pour appliquer des forces de pression sur la face externe de la membrane,
des moyens de mesure des contraintes radiales et axiales auxquelles est soumis l'échantillon et
des moyens de mesure des déformations subies par l'échantillon.

D'autres avantages et caractéristiques de l'invention ressortiront à la lecture de la description suivante faite à titre d'exemple et en référence aux dessins annexés dans lesquels :
la figure 1 est une vue en coupe selon un plan axial vertical d'un dispositif selon l'invention au cours d'une mesure in situ ;
la figure 2 est une vue schématique et en coupe du dispositif de la figure 1, au cours de son enfoncement dans le sol, qui montre les moyens de désagrégation du sol à la sortie du carottier ; et
la figure 3 est une vue schématique et en coupe d'une variante de réalisation du dispositif.

Le corps creux 1 de la sonde comporte sur sa face intérieure un rétréci servant au logement d'une membrane souple et dilatable 2, qui, en l'absence de forces de pression sur sa face externe, se trouve dans le prolongement de la paroi interne 3 d'une trousse coupante 4 fixée sur l'extrémité inférieure du corps creux 1, au moyen d'un filetage 6. La trousse coupante 4 peut ainsi être remplacée en cas d'usure de son extrémité inférieure 7 qui présente une section biseautée.

L'extrémité supérieure du corps creux 1 est reliée à une tête d'outil 8 qui comporte des moyens de fixation 9 sur l'extrémité inférieure d'une tige 10 de vérinage actionnée par des moyens appropriés disposés à la surface du sol à tester.

Lorsqu'on exerce sur la tige 10 une force verticale F, la trousse coupante 4 s'enfonce dans le sol 11 à la manière d'un carottier et un échantillon 12 de sol pénètre dans la cavité de la trousse coupante 4 et du corps creux 1.

Dans la paroi interne du corps creux 1, est ménagé un passage 13 qui débouche légèrement au-dessus du plan supérieur P1 de la membrane 2, par un orifice 14 dans lequel est monté un système de lançage alimenté sous haute pression par un coulis de bentonite, par exemple, ce qui permet de désagréger la carotte de sol qui sort de la zone de mesure. Le passage 13 est relié à la surface par un conduit 15 qui délivre le coulis.

La chambre 16, ménagée entre la membrane 2 et la face interne du corps creux, peut être alimentée par un fluide sous pression délivrée à partir de la surface grâce à un conduit 17 et à un passage 18 ménagé dans le corps creux 1. Cette chambre 16 comporte en outre des moyens de détection 19 du déplacement de la membrane 2 et des moyens de mesure de la pression, ces moyens étant reliés à la surface par des câbles.

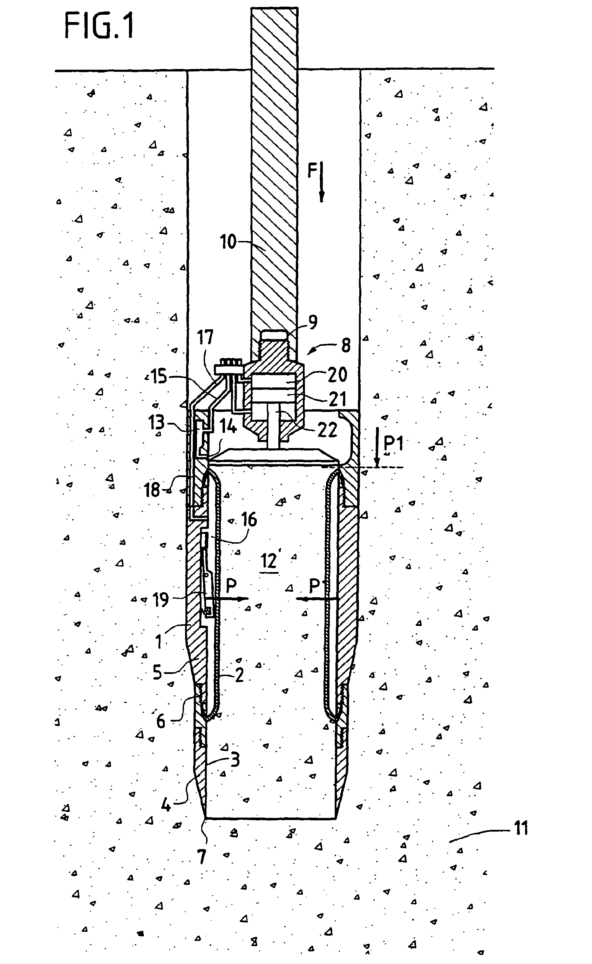

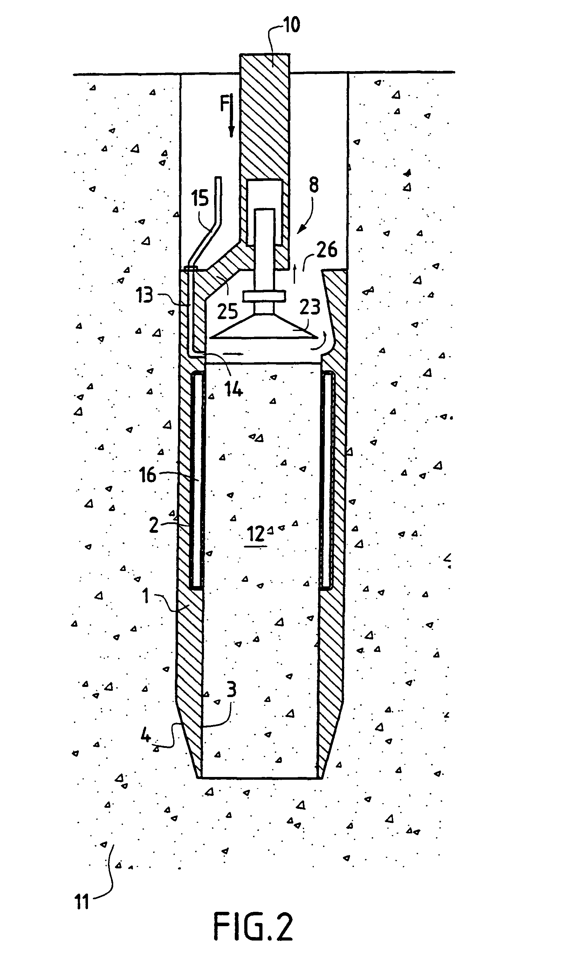

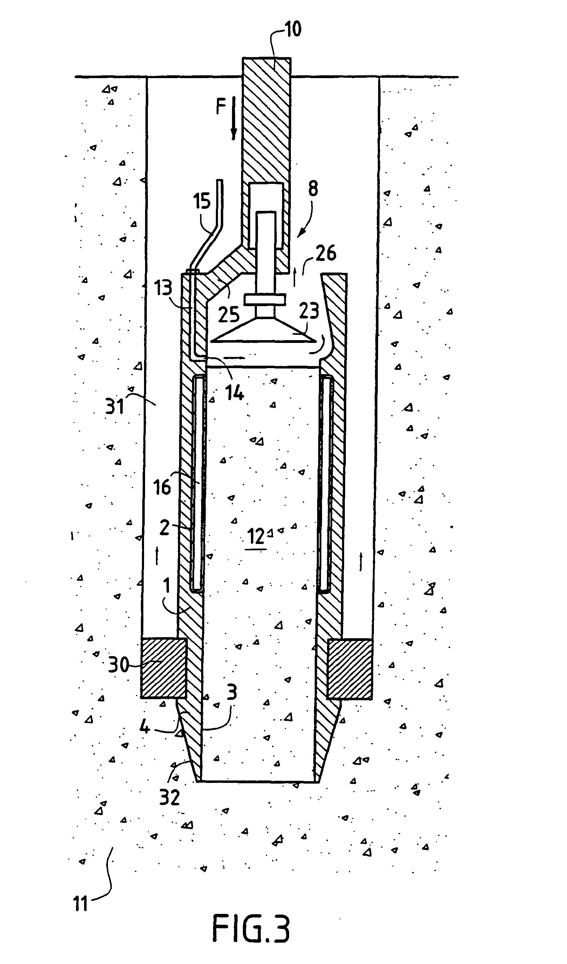

La tête d'outil 8 comporte une cavité cylindrique 20 d'axe vertical, dans laquelle est monté un piston de manoeuvre 21 à double effet dont la tige de piston 22 traverse la face inférieure de la tête d'outil 8. L'extrémité inférieure de la tige de piston est équipée d'un deuxième piston 23, dont le diamètre est sensiblement égal au diamètre du corps creux 1. La déplacement vertical du piston de manoeuvre 21 entraîne le déplacement vertical du deuxième piston 23. Ce deuxième piston peut prendre une position haute montrée sur les figures 2 et 3, située au-dessus du plan de l'orifice 14, utilisée lors de l'enfoncement de la sonde, et une position basse, montrée sur la figure 1, dans laquelle la face inférieure est sensiblement au niveau du plan P1 et peut exercer une force sur la face supérieure de l'échantillon 12 emprisonné dans la membrane 2 et dans la trousse coupante 4. Cette position basse est utilisée au cours d'une mesure, et la force est générée par le piston de manoeuvre 21. Les deux chambres de la cavité cylindrique 20, délimitées par le piston de manoeuvre 23, sont reliées à un circuit hydraulique commandé depuis la surface et non montré sur les dessins.

La tête d'outil 8 est reliée au corps creux 1 par des bras 25 qui ménagent entre eux des passages 26 par lesquels le mélange de coulis et de sol désagrégé par le système de lançage remonte vers la surface.

Pour réaliser une mesure, on procède de la manière suivante :

Au cours de la phase d'enfoncement de la sonde dans le sol, la chambre 16 est mise hors pression, le deuxième piston 23 est en position haute, et le système de lançage est mis en fonctionnement. On enfonce la sonde dans le sol grâce à la tige 10 de vérinage d'une profondeur au moins égale à la hauteur de la sonde. L'échantillon 12 de la mesure précédente est désagrégé par le système de lançage et un nouvel échantillon 12 se forme à l'intérieur de la membrane 2.

A la fin de cette opération d'enfoncement, on immobilise la tige 10 de vérinage, on arrête le système de lançage, on applique le deuxième piston 23 sur la face supérieure de l'échantillon 12, avec une force faible.

On procède ensuite à la mesure proprement dite. Pour cela, on met la chambre 16 à une pression prédéterminée P. L'échantillon 12 est ainsi soumis à des contraintes radiales prédéterminées. On fait ensuite varier la contrainte axiale de l'échantillon 12, en faisant varier la force exercée sur la face supérieure de l'échantillon 12 au moyen du piston de manoeuvre 21. Pendant l'essai, la contrainte radiale est maintenue constante et c'est la contrainte axiale qui croît jusqu'à la rupture de l'échantillon 12 par cisaillement.

De par sa conception, le dispositif proposé permet d'imposer des chemins de contraintes. Ceci est fait par l'asservissement du deuxième piston 23 et de la chambre 16 de pression latérale. La réalisation de mesures locales permet d'atteindre le domaine des petites déformations. Les mesures locales sont obtenues en plaçant les capteurs au plus près de l'échantillon 12, c'est-à-dire que des capteurs de déplacements, radiaux et axiaux, ainsi qu'un capteur de pression interstitielle, sont fixés sur la membrane 2.

Dans le mode de réalisation décrit ci-dessus, le corps creux 1, la trousse coupante 4 et le deuxième piston 23 ont une section circulaire.

Le corps creux 1, la trousse coupante 4 et le deuxième piston 23 peuvent avoir des sections carrées. Un tel dispositif permet de réaliser un vrai test triaxial, et de déterminer les paramètres d'anisotropie de l'échantillon 12 testé.

La figure 3 montre une variante de réalisation, de type cylindrique, dans laquelle le corps de sonde 1 comporte à l'extérieur et en retrait de la trousse coupante 4 un outil désagrégateur 30 qui forme un puits 31. Cette variante de réalisation permet de tester des matériaux de nature variée et difficilement prélevables, ainsi que des matériaux assez grossiers.

Le biseau de la trousse coupante 4 est tel que la face interne 3 de l'extrémité inférieure de la trousse 4 est cylindrique, tandis que la face externe 32 est conique et provoque le refoulement du sol non testé vers l'extérieur de la sonde au cours de l'enfoncement de la sonde. La face interne 3 provoque un lissage latéral de l'échantillon 12 dont l'influence sur les mesures est négligeable.

Le dispositif selon l'invention permet de réaliser un test voisin du test triaxial de révolution réalisé en laboratoire. Mais le rendement est nettement accru du fait que les essais sont réalisés pas à pas de façon quasi continue et sans manoeuvres importantes.

## Revendications

1. Procédé pour mesurer in situ les modules de déformation d'un échantillon (12) de sol dans des conditions voisines d'un essai triaxial, **caractérisé par** les étapes suivantes :
on enfonce dans le sol (11) un carottier (1, 4) équipé, sur sa face interne, d'une membrane (2) souple,
on évacue les matériaux qui ressortent par l'orifice supérieur du carottier (1, 4) au cours de son enfoncement, afin de former un échantillon (12) de sol à tester dans le carottier,
on immobilise le carottier (1, 4) dans le sol pour procéder à une mesure,
on exerce des forces de pression sur la face externe de la membrane (2) et sur la face supérieure de l'échantillon (12), afin de soumettre l'échantillon à des contraintes radiales et axiales, et
on mesure les déformations subies par l'échantillon (12).

2. Procédé selon la revendication 1, **caractérisé par le fait que**, pendant la mesure, on soumet l'échantillon (12) à des contraintes radiales prédéterminées, et on fait varier les contraintes axiales jusqu'à la rupture de l'échantillon (12).

3. Procédé selon l'une des revendications 1 ou 2, **caractérisé par le fait qu'**on utilise un carottier (1, 4) ayant une section interne sensiblement carrée.

4. Dispositif pour mesurer in situ les modules de déformation d'un sol dans les conditions d'un essai triaxial, **caractérisé par le fait qu'**il comporte :
un carottier (1, 4) équipé sur sa face interne d'une membrane (2) souple,
des moyens (10) pour enfoncer ledit carottier dans le sol (11) afin de former un échantillon (12) dans ledit carottier,
des moyens pour évacuer les matériaux qui ressortent par l'orifice supérieur dudit carottier au cours de son enfoncement dans le sol (11),
des moyens pour immobiliser ledit carottier au cours de la mesure,
des moyens pour appliquer une force axiale sur la face supérieure de l'échantillon (12),
des moyens pour appliquer une pression sur la face externe de la membrane (2) souple,
des moyens de mesure des contraintes radiales et axiales auxquelles est soumis l'échantillon (12), et
des moyens de mesure des déformations subies par l'échantillon (12).

## Claims

1. A method of measuring in situ the deformation moduluses of a soil sample (12) under conditions close to those of a triaxial compression test, the method being **characterised by** the following steps:
driving a corer (1, 4) into the soil (11), the corer being fitted on its inside face with a flexible diaphragm (2);
removing the material that leaves the corer (1, 4) through its top orifice as it is being driven into the soil, so as to form a sample (12) of soil for testing inside the corer;
holding the corer (1, 4) stationary in the soil in order to proceed with a measurement;
exerting pressure forces on the outside face of the diaphragm (2) and on the top face of the sample (12) so as to subject the sample to radial and axial stresses; and
measuring the deformations to which the sample (12) is subjected.

2. A method according to claim 1, **characterised by** the fact that during measurement, the sample (12) is subjected to predetermined radial stresses, and the axial stresses are varied until the sample (12) breaks.

3. A method according to claim 1 or claim 2, **characterised by** the fact that a corer (1, 4) is used having an inside section that is substantially square.

4. Apparatus for measuring in situ the deformation moduluses of soil under the conditions of a triaxial compression test, the apparatus being **characterised by** the fact that it comprises:
a corer (1, 4) fitted on its inside face with a flexible diaphragm (2);
means (10) for driving said corer into the soil (11) in order to form a sample (12) inside said corer;
means for removing the material which leaves said corer through its top orifice while the corer is being driven into the soil (11);
means for holding said corer stationary while taking a measurement;
means for applying an axial force to the top face of the sample (12);
means for applying pressure to the outside face of the flexible diaphragm (2);
means for measuring the radial and axial stresses to which the sample (12) is subjected; and
means for measuring the deformations to which the sample (12) is subjected.

## Patentansprüche

1. Verfahren zum Messen in situ der Deformationsmodule einer Bodenprobe (12) unter Bedingungen, die denen eines Dreiachsentests nahe sind, das durch die folgenden Schritte **gekennzeichnet ist:**
man treibt in den Boden (11) einen Kernbohrer (1, 4), der auf seiner Innenseite mit einer elastischen Membran (2) ausgestattet ist, man beseitigt die Materialien, die durch die obere Öffnung des Kernbohrers (1, 4), während seines Eintreibens, treten, um eine Probe (12) des zu testenden Bodens in dem Kernbohrer zu bilden man bringt den Kernbohrer (1, 4) im Boden zum Stehen, um eine Messung vorzunehmen,
man übt Druckkräfte auf die Außenseite der Membran (2) und die Oberseite der Probe (12) aus, um die Probe radialen und axialen Belastungen zu unterziehen, und
man misst die der Probe (12) zugefügten Verformungen.

2. Verfahren nach Anspruch 1, **dadurch gekennzeichnet, dass** man während der Messung die Probe (12) vorbestimmten radialen Belastungen unterzieht und man die axialen Belastungen bis zum Brechen der Probe (12) variieren lässt.

3. Verfahren nach einem der Ansprüche 1 oder 2, **dadurch gekennzeichnet, dass** man einen Kernbohrer (1, 4) mit einem im Wesentlichen viereckigen inneren Querschnitt verwendet.

4. Vorrichtung zum Messen in situ der Deformationsmodule eines Bodens (12) unter den Bedingungen eines Dreiachsentests, **dadurch gekennzeichnet, dass** sie aufweist:
einen Kernbohrer (1, 4), der auf seiner Innenseite mit einer elastischen Membran (2) ausgestattet ist,
Mittel (10) zum Eintreiben des Kernbohrers in den Boden (11), um eine Probe (12) in dem Kernbohrer zu bilden,
Mittel zum Beseitigen der Materialien, die durch die obere Öffnung des Kernbohrers, während seines Eintreibens in den Boden (11) treten,
Mittel, um den Kernbohrer während der Messung zum Stehen zu bringen,
Mittel, um eine axiale Kraft auf die Oberseite der Probe (12) anzuwenden,
Mittel zum Anwenden eines Drucks auf die Außenseite der elastischen Membran (2),
Mittel zum Messen der radialen und axialen Belastungen, denen die Probe (12) unterzogen wird, und
Mittel zum Messen der der Probe (12) zugefügten Verformungen.
